# EUROPEAN PATENT SPECIFICATION

(11) **EP 1 908 421 B1**
(45) Date of publication and mention of the grant of the patent: **26.08.2009**
(21) Application number: 06021065.5
(22) Date of filing: 06.10.2006
(51) Int. Cl.: A61B 17/11, A61B 17/22

(54) **A carrier member, anastomotic device and instrumentation for performing endoluminal and/or transluminal anastomosis**
Trägerelement, Anastomosevorrichtung und Instrumente zur Durchführung einer endoluminalen und/oder transluminalen Anastomose
Membre de support, dispositif d'anastomose et instrumentation pour effectuer une anastomose endoluminale et/ou transluminale

(43) Date of publication of application: 09.04.2008
(73) Proprietor: ETHICON ENDO-SURGERY, INC., Cincinnati, OH 45242-2839 (US)
(72) Inventor: Pastorelli, Alessandro, 00136 Roma (IT)
(74) Representative: Leihkauf, Steffen Falk

(56) References cited:
- EP-A1- 0 517 488
- WO-A1-20/06048904
- US-A1- 2006 224 166

## Description

The present invention relates, in general, to devices for surgically modifying organs and vessels and more particularly to a surgical instrument for performing an endoluminal and or transluminal anastomosis, particularly of the digestive tract, such as gastro-jejunostomy, jejuno-jejunostomy or similar interventions as for example colo-proctostomy, jejuno-colostomy or anastomoses involving the Chole duct, by applying an anastomotic ring device comprising two snap-connectable rings.

The known surgical methods and instruments for performing the above mentioned anastomoses by applying anastomotic ring devices involve traditional open surgery or laparoscopic surgical techniques, which are rather invasive and require the use of quite complex and cumbersome surgical devices. As a result the risk of post-operative complications is undesirably high.

Moreover, the known devices and methods are not suitable to perform the anastomosis by a pure or at least partially endoscopic or endolumenal approach.

Such an endolumenal approach, if assisted by a suitable instrumentation, would sensibly reduce the disadvantages of the traditional open surgery and laparoscopic methods. Particularly, endolumenal operations would reduce the invasiveness of the intervention, thereby reducing the risk of complications and shortening the post operative course of the patient.

Even though the endoscopic and endolumenal approach to anastomosis is very promising, it is presently very difficult to use such an approach in practice, e.g. for performing an anastomosis of the digestive tract, since neither the instrumentation nor the methods are mature enough to assure for instance an adequate transportation of the ring device to the anastomotic side, a controlled tissue approximation and a reliable alignment and connection of the rings during deployment. As a result, operative and post operative complications cannot be excluded.

WO 2006/048904 A1 discloses an anastomosis device having features of the preamble of annexed claim 1.

The object of the present invention is therefore to provide a surgical instrumentation, particularly a carrier member and an anastomotic device, especially developed and adapted for performing endoluminal or transluminal anastomoses, particularly of the digestive tract, by an endolumenal approach.

There is provided an exemplary surgical method for performing an endolumenal or translumenal anastomosis, particularly of the digestive tract, by an endolumenal or endoscopic approach which is less invasive than the currently employed open surgery and laparoscopic approaches.

These and other objects are achieved by an anastomosis device according to claim 1. Advantageous embodiments are claimed in the dependent claims.

For better understanding the invention and appreciating the related advantages, a detailed and non limiting description of embodiments is provided with reference to the accompanying drawings, in which:
- Figure 1 is a perspective partially sectioned view of a portion of the stomach and of the small intestine subject to a step of an exemplary method for creating a guide wire loop;
- Figures 2 to 14 illustrate subsequent steps of the exemplary method, as well as an endoscope and guide wire means for creating the guide wire loop;
- Figure 15 is a perspective view of a carrier part and a proximal ring of an anastomotic device according to the invention involved in a step of an exemplary method for delivering the proximal ring to a proximal tissue portion;
- Figures 16 to 19 illustrate subsequent steps of the exemplary method for delivering the proximal ring,
- Figure 20 is a perspective view of the anastomotic device and of an anastomotic applier in a step of an exemplary method for delivering the distal ring to a distal tissue portion and connect the distal and proximal rings;
- Figures 21 to 23 illustrate subsequent steps of the exemplary method for connecting the anastomotic rings;
- Figures 24 to 26 illustrate the anastomotic device and applier during subsequent steps of the exemplary method in which the instrumentation is removed from the body and a gastro-jejunostomy is completed;
- Figure 27 is a perspective view of the portion of the stomach and of the small intestine from figure 1 after completion of the gastro-jejunostomy;
- Figures 28 and 29 illustrate two subsequent steps of an exemplary method for performing anastomoses in segments of the digestive tract;
- Figure 30 is an exploded perspective view of the anastomotic device according to an embodiment of the invention;
- Figure 31 is a perspective view of a carrier part of the anastomotic device according to a further embodiment of the invention.

For the sake of clarity and for better evidencing the technical effect of the features of the anastomotic device according to the invention and its interaction with the particular environment of application, the following detailed description of the invention will first deal with the exemplary surgical method thought up by the inventors and subsequently describe the anastomotic device and surgical instruments for carrying out the exemplary method.

### Overall procedure to perform the anastomosis

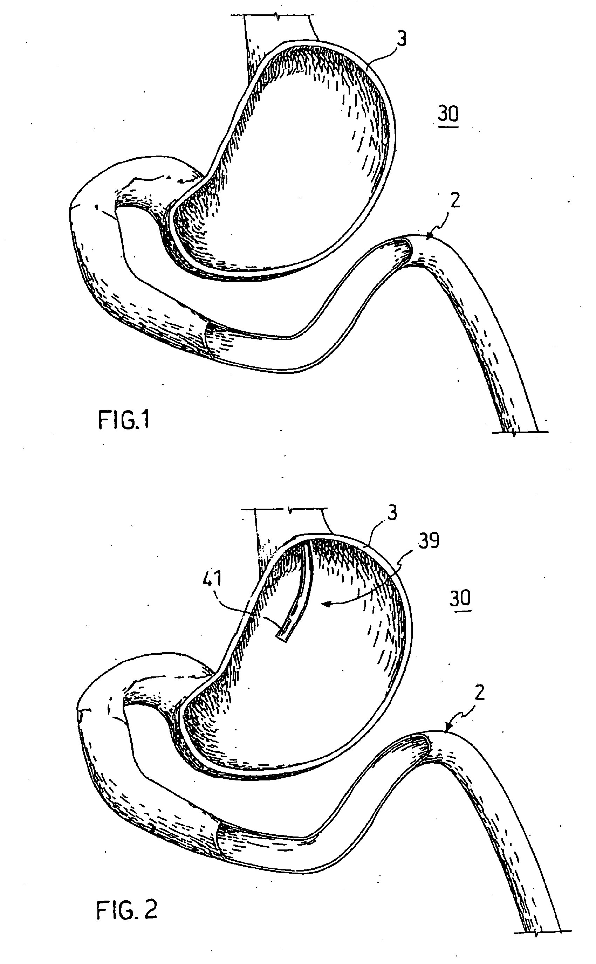

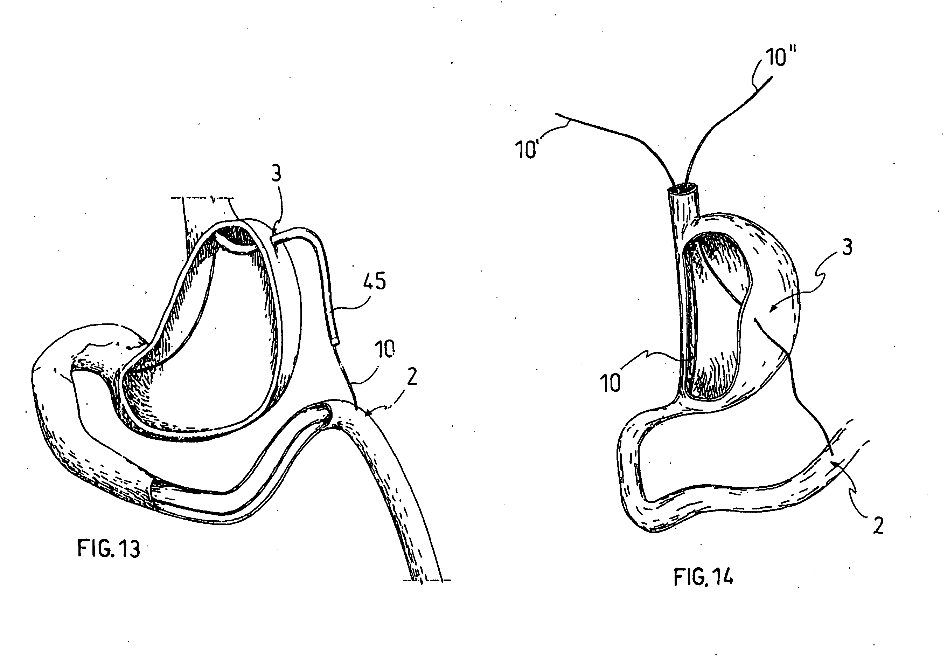

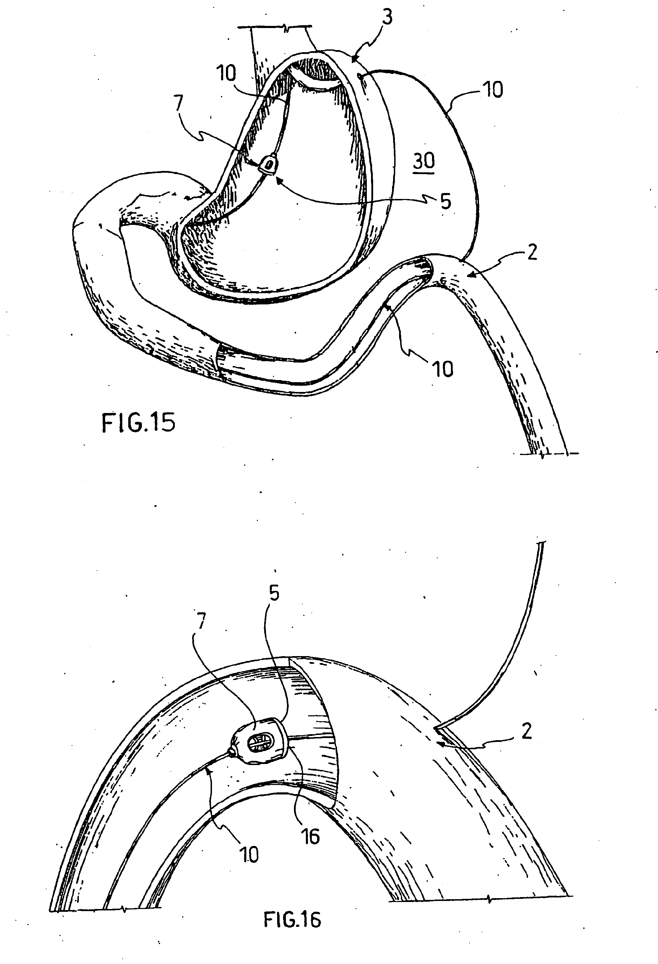

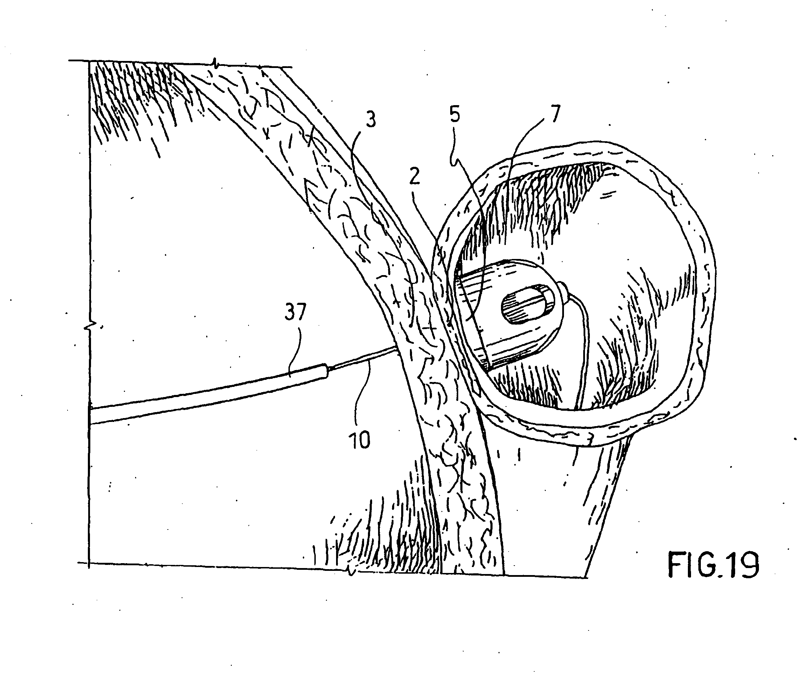

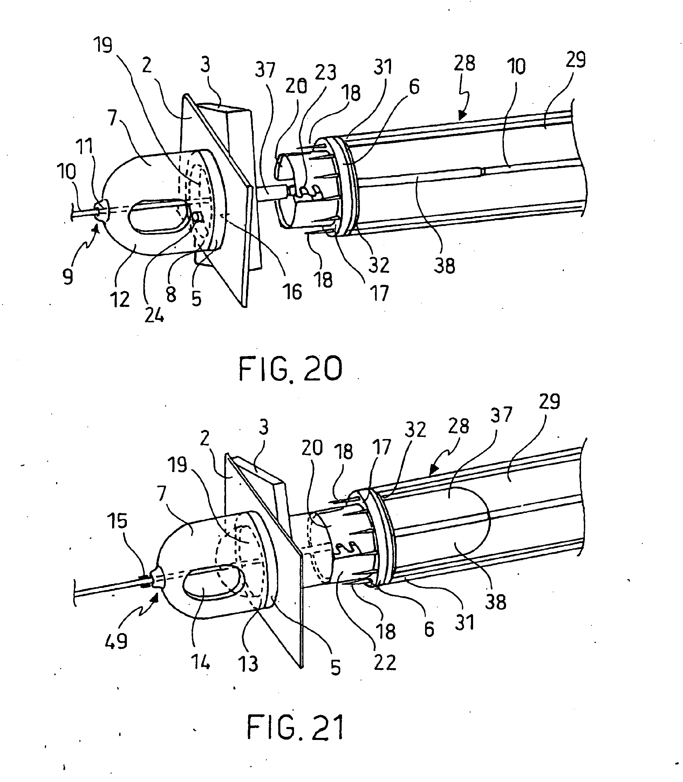

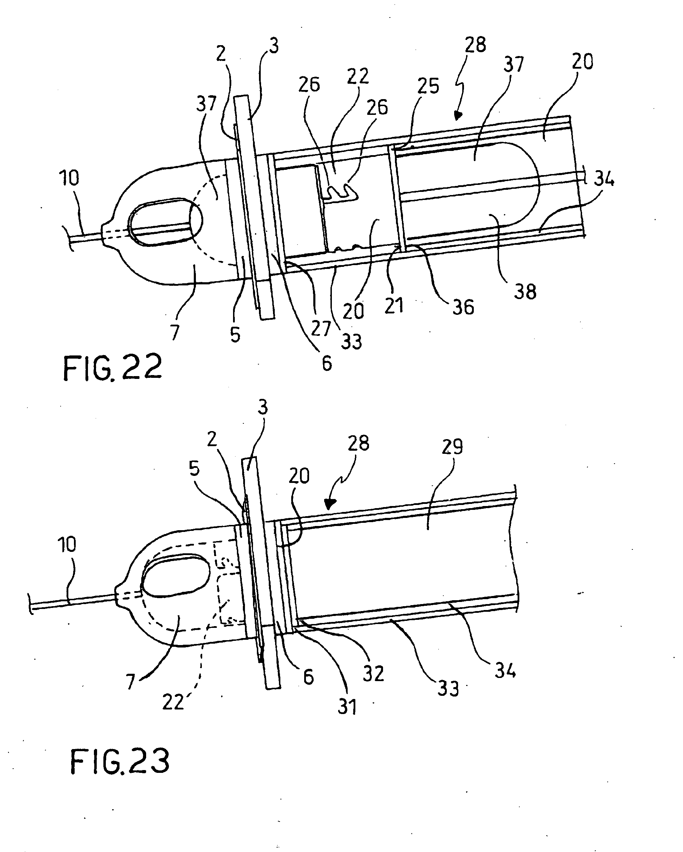

An exemplary method for performing an endoluminal or transluminal anstomosis, such as e.g. a gastro-jejunostomy, a jejuno-jejunostomy, a colo-proctostomy, a jejuno-colostomy or anastomoses of the chole duct, comprises generally the following steps:
- Creating a loop of guide wire means by placing guide wire means 10 in the body of a patient in a way that the guide wire means 10 extend from an extracorporeal proximal end 10' into the body where it goes through a proximal tissue portion 2 and through a distal tissue portion 3 which are planned to be joined in anastomosis and out of the body up to an extracorporeal distal end 10'' (Fig. 2 - 14). In the following, if not otherwise specified, the terms "proximal" and "distal" are referred to the directions along the guide wire loop and to the above defined proximal and distal ends thereof.
- Fixing a proximal ring 5 of an anastomotic ring device to the proximal end 10' of the guide wire means 10 and delivering the proximal ring 5 to the proximal tissue portion 2 by pulling the distal extracorporeal end 10" of the guide wire means 10 in a distal direction until the proximal ring 5 reaches the proximal tissue portion 2 (Fig. 15 - 18),
- Slidably connecting a distal ring 6 of the anastomotic ring device to the distal end 10'' of the guide wire means 10 and pushing it proximally along the guide wire means until it reaches the distal tissue portion 3 (Fig. 19 - 21),
- Contemporaneously pulling the distal end 10" of the guide wire means 10 distally and pushing the distal ring 6 proximally to approximate the proximal and distal rings, thereby tearing the proximal and distal tissue portions 2, 3 situated upon the guide wire means between the distal and proximal rings 5, 6 in compression contact to another (Fig. 21, 22),
- Connecting the distal ring 6 with the proximal ring 5, thereby clamping the distal and proximal tissue portions 2, 3 between them (Fig. 23),
- Widening the tissue internally overhanging the anastomotic ring device to open the anastomotic lumen (Fig. 20, 21),
- Pulling the proximal end 10' of the guide wire means 10 to remove the guide wire means 10 from the body.

The loop of the guide wire means 10 starts and ends either in natural orifices, like mouth, nose, anus or, alternatively, in artificially created openings in the body, such as colostomy, abdominal incisions, wound or fistulas. Preferably, the guide wire means 10 enters and exits the body through natural ducts (e.g. mouth).

While the guide wire means can comprise one or more single flexible guide wires, it is preferable to provide only one single guide wire 10 which penetrates the proximal and distal tissue portion.

### Creation of the loop of the guide wire means

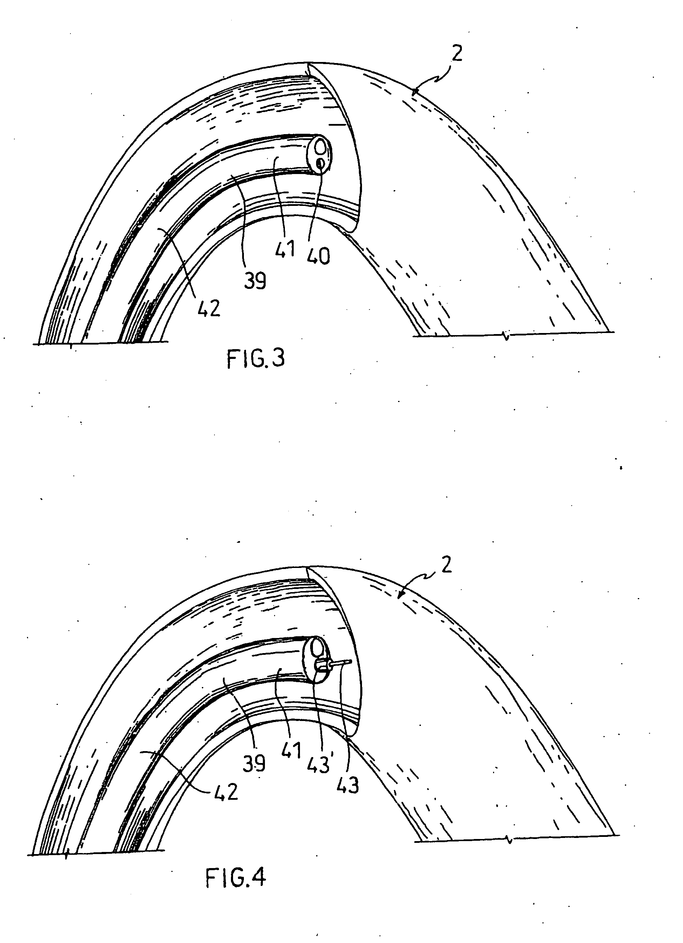

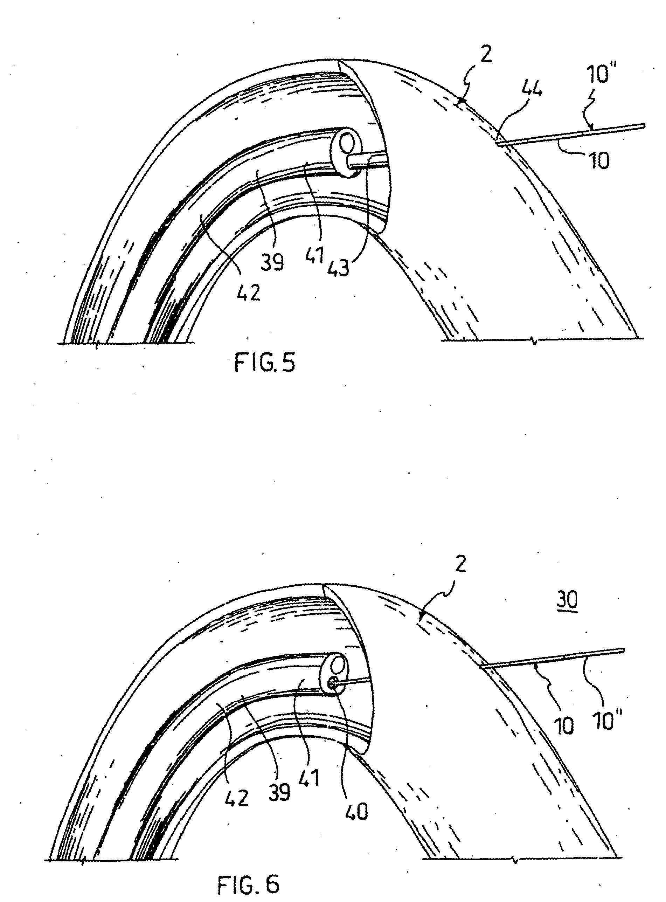

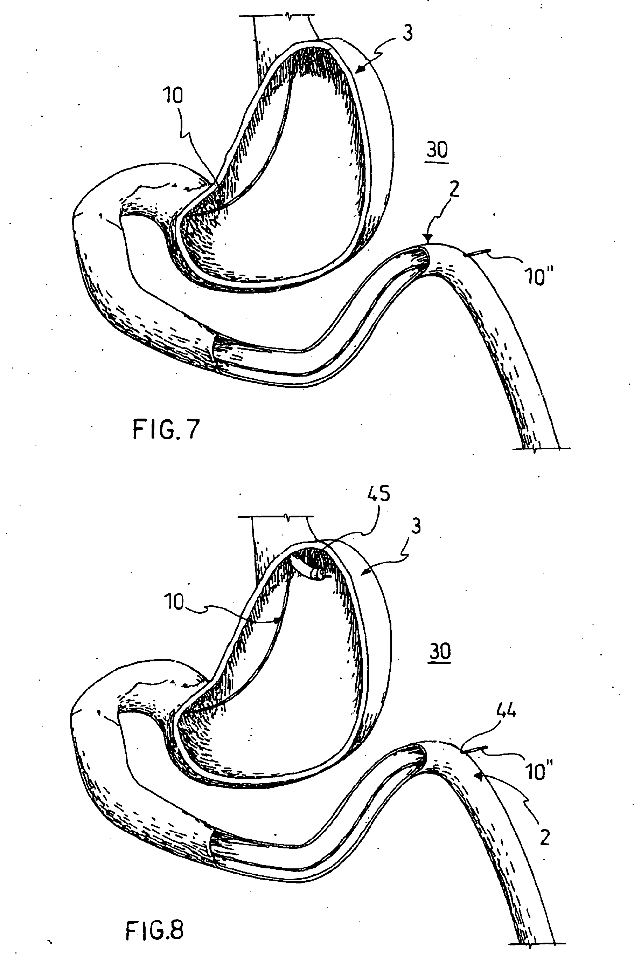

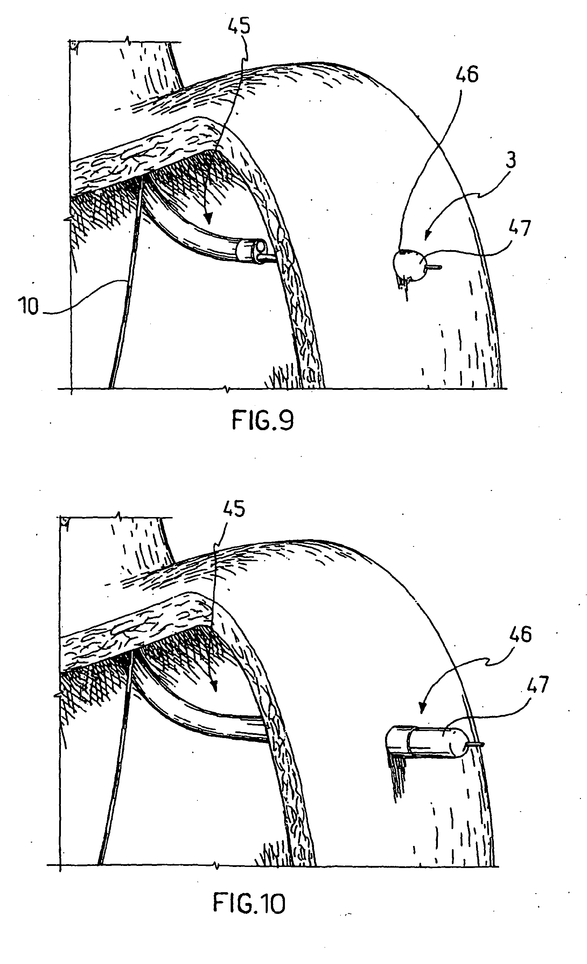

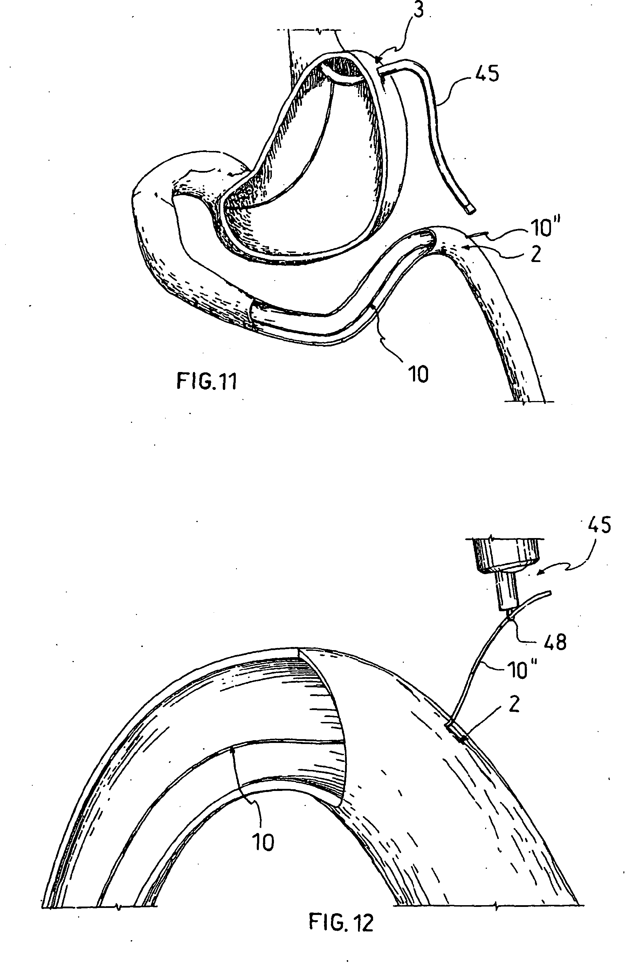

The loop of the guide wire means 10 is created by means of the following procedural steps:
- Transluminally (e.g. transorally) introducing an elongate insertion device 39 through the proximal inlet port (e.g. mouth) for the guide wire means 10 and pushing the insertion device 39 from outside the body distally towards the proximal tissue portion 2 (e.g. a jejunal anstomotic site),
- Transporting the distal end 10'' of the guide wire means 10 to the proximal tissue portion 2 through one or more guide wire canals 40 formed in the insertion device 39 and perforating the proximal tissue portion 2 with the guide wire end 10'' or needle guide wire end. Alternatively, the proximal tissue portion 2 is perforated by a distinct radio frequency needle device (Fig. 4) having a sheath 43' which is insertable along the guide wire canal 40 and which defines two or more internal canals (one for the RF needle 43 and one for the guide wire 10).
   In this way the distal guide wire end 10'' protrudes distally from the proximal tissue portion 2 (e.g. into the previously CO₂ insufflated abdominal space 30),
- Removing the insertion device 39 from the body by pulling it proximally out of the proximal inlet port (e.g. mouth) and leaving the guide wire means 10 in place (Fig. 6 - 7),
- Transluminally introducing a grasping device 45 through the distal inlet port for the guide wire means which might but need not coincide with the proximal inlet port (e.g. mouth) and pushing the grasping device 45 from outside the body proximally (with reference to the loop direction) towards the distal tissue portion 3, e.g. the gastric wall tissue (Fig. 8, 9),
- Creating a hole 46 in the distal tissue portion 3, e.g. by means of a radiofrequency needle or equivalent penetration devices and, preferably, widening the hole 46 with a balloon catheter 47 which is preferably transported to the distal tissue portion 3 through a working channel of the grasping device 45 (Fig. 9, 10).
- Passing the grasping device 45 through the previously widened hole (gastrotomy) 46 of the distal tissue portion 3 into the same space where the distal guide wire end 10'' lays (e.g. in the previously CO₂ insufflated abdominal space 30) and pushing it towards the distal end 10" of the guide wire means 10 (Fig. 11).
- Inserting a snare 48 through the working or instrument delivery canal formed in the grasping device 45 and advancing the snare 48 proximally until it exits from the grasping device 45 and reaches the distal guide wire end 10".
- Feeding the snare 48 over the distal guide wire end 10" and closing or tightening it around the guide wire means 10 in order to catch it, subsequently pulling the snare 48 together with the distal end 10'' of the guide wire means 10 distally through the perforation 46 of the distal tissue portion 3 (e.g. gastric wall) and distally withdrawing the grasping device 45 together with the distal guide wire end 10" through the distal inlet port (e.g. transorally) out of the body (Fig. 12 -14).

Advantageously, the transluminal introduction of the grasping device 45 and the snare 48 is performed by a pure endoscopic or endolumenal approach. Alternatively, this procedural step is performed under laparoscopic supervision. In this case the hole 46 does not need to be widened and only the snare 48 (preferably a radio frequency snare adapted to pierce through the gastric wall) creates the hole and is passed through it into the abdominal space 30. The distal guide wire end 10" is then laparoscopically fed into the snare hole to be caught by the latter.

In case of a gastro-jejunostomy, the insertion device 39 needs to be advanced transorally through the esophagus and the stomach and across the pylorus into the duodenum, which is not always easy to point at. During this step of the procedure an Ewald tube might be pushed through the patients mouth down the esophagus into the stomach and the insertion device 39 can be advantageously guided inside the Ewald tube up to and across the pylorus.

### Delivering the proximal ring of the anastomotic ring device to the anastomotic site and approximation of the proximal and distal tissue portions

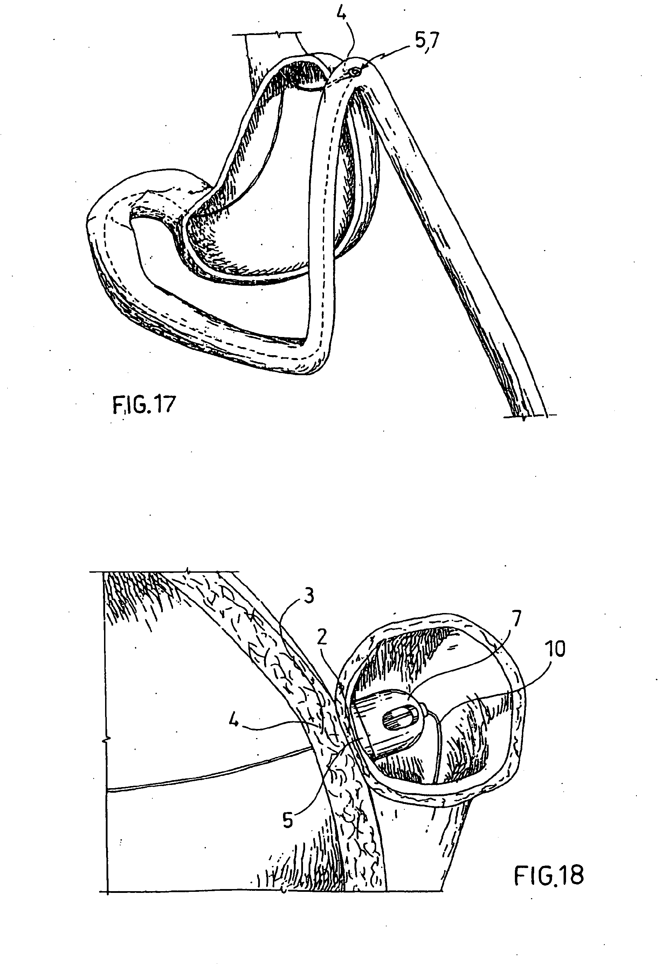

The proximal ring is delivered to the anastomotic site 4 by means of the following procedural steps:
- Detachably connecting the proximal ring 5 with a, preferably distinct, carrier member 7 outside the body of the patient.
- Extracorporeal attachment of the carrier member 7 (which holds the proximal ring 5) to the proximal end 10' of the guide wire means, e.g. by inserting it over the single guide wire 10, and locking the carrier member 7 in a manner to prevent it from sliding along the guide wire means 10. The carrier member 7 is preferably locked by means of locking elements 15, such as clips which can be clamped around the guide wire 10 and which are adapted to provide an obstacle against sliding of the carrier member 7. After having connected the carrier member 7 to the proximal guide wire end 10', it is introduced in the proximal endoscopic inlet port and pulled to the anastomotic site 4, i.e. to the proximal tissue portion 2 (e.g. intestinal wall) by pulling the distal guide wire end 10'' distally (Fig. 15-17)
   Alternatively, the locking of the carrier member 7 to the guide wire 10 might be obtained by locking features, for instance clamping portions, integrated in the carrier member 7. According to a yet further embodiment, the carrier member 7 has been fabricated and distrubuted together with the guide wire or previously connected to it in a manner that, after completion of the guide wire loop, it is sufficient to introduce the carrier member 7 together with the proximal ring 5 into the proximal inlet port (e.g. mouth and esophagus) and deliver it to the proximal tissue portion 2 by pulling the distal guide wire end 10" distally.
- Subsequently, approximating the proximal tissue portion 2 (e.g. intestinal tissue) towards the distal tissue portion 3 (e.g. gastric wall) until they are in intimate contact by pulling the distal guide wire end 10" distally so that the carrier member 7 urges the proximal ring 5, particularly a distal tissue contact surface 16 thereof, against the proximal tissue portion 2 and moves the latter distally towards and in contact with the distal tissue portion 3 (Fig. 18). In other words, Fig. 15 to 18 illustrate an advantageous use of the anastomotic device 1, i.e. the approximation of two tissue portions intended to be joined in anastomosis.

### Delivering the distal ring of the anastomotic ring device to the anastomotic site and connection of the distal and proximal rings

The proximal ring is delivered to the anastomotic site 4 by means of the following procedural steps:
- Inserting the distal compression ring 6 over the distal guide wire end 10'' and pushing it proximally along the guide wire means 10 towards the anastomotic site 4 and, hence, towards the proximal compression ring 5. Thanks to the guide wire loop, the distal and proximal compression rings, together with the distal and proximal tissue portions are axially aligned in the anastomotic site 4. The distal compression ring 6 is pushed along the guide wire 10 by means of an apposite anastomotic applier 28 which is advantageously adapted to perform an angular positioning, if required, of the distal compression ring 6 with respect to the proximal compression ring 5 in order to enable their mutual connection. Alternatively, the compression rings are provided with connecting features which enable their mutual connection in different angular positions or independently from their reciprocal angular position.
- Connecting the compression rings 5, 6 and, hence the tissue portions 2, 3 clamped between them, by pushing the distal compression ring 6 proximally in engagement with the proximal compression ring 5, while the latter is held in position by counter-pulling the distal guide wire end 10'' distally, and successively or contemporaneously connecting the compression rings 5, 6 to another.

The compression rings 5, 6 are connected by applying a distinct locking device 20 adapted to engage both compression rings and to maintain them connected at a desired reciprocal distance. Advantageously, the distal compression ring 6 is firstly pushed proximally against the proximal compression ring 5 and successively the locking device 20 is pushed in connecting engagement with both the distal and proximal compression rings 5, 6 to connect them.

The exemplary method of connecting the compression rings to another can additionally comprise the step of fixating the tissue portions 2, 3 to the compression rings 5, 6 in order to effectively prevent the tissue from radially slipping out of the grip engagement with the compression rings. This additional step might be performed before or contemporaneously with the application of the locking device 20 (Fig. 21, 22). This additional fixation step is preferably performed by compressing the tissue portions 2, 3 between the distal 6 and proximal ring device 5 and by driving a plurality of needles 18 into and across the ring device and the tissue portions 2, 3 so that the needles 18 provide the above said additional fixation (Fig. 21, 22).

As the delivery of the locking device 20 and the group of needles 18 (if provided) to the anastomotic site 4 is concerned, the locking device 20 is inserted extracorporeal over the distal guide wire end 10" and pushed proximally along the guide wire 10 towards the anastomotic site 4 and towards the proximal compression ring 5. Preferably, both the distal compression ring 6 and the distinct locking device 20 (and, if provided, the needle group 18) are arranged on a distal end (here the term "distal" is referred to the surgeons viewpoint and not to the guide wire loop) of the anastomotic applier 28 and pushed by means of the latter to the anastomotic site 4 and in engagement with the proximal compression ring 5 (Fig. 20-23).

### Widening the tissue at the anastomotic site

The distal and proximal tissue portions intended to be joined in anastomosis are widened by means of the following procedural steps:
- widening the passage apertures 44, 46 of the tissue portions 2, 3 through which the guide wire means, preferably a single guide wire 10, passes by cutting or forced tissue extension after the tissue portions 2, 3 have been approximated in mutual contact but, preferably, before the distal and proximal compression rings are definitely connected. The passage openings of the tissue portions are widened by an endoscopic balloon dilator 37 which is extracorporeally inserted over the distal guide wire end 10" and proximally pushed along the guide wire 10 towards and at least partially across the passage openings 44, 46 in the tissue portions 2, 3 and into the distal cavity of the carrier member 7. Successively, the endoscopic balloon dilator 37 is insufflated to an extent that it fills approximately a central aperture 19 of the proximal compression ring 5, thereby widening the anastomotic lumen to substantially the same extent (Fig. 20, 21).

Prior to or after the inflation of the balloon dilator 37 the distal compression ring 6 (together with the needle group if provided) and the locking device 20 are inserted over a proximal portion 38 of the balloon dilator 37 (referred to the surgeons point of view) such that the inflated balloon dilator 37 provides a guide for the successive approximation and alignment of the compression rings 5, 6 (Fig. 21, 22).

### Removing the instrumentation and guide wire means from the body of the patient

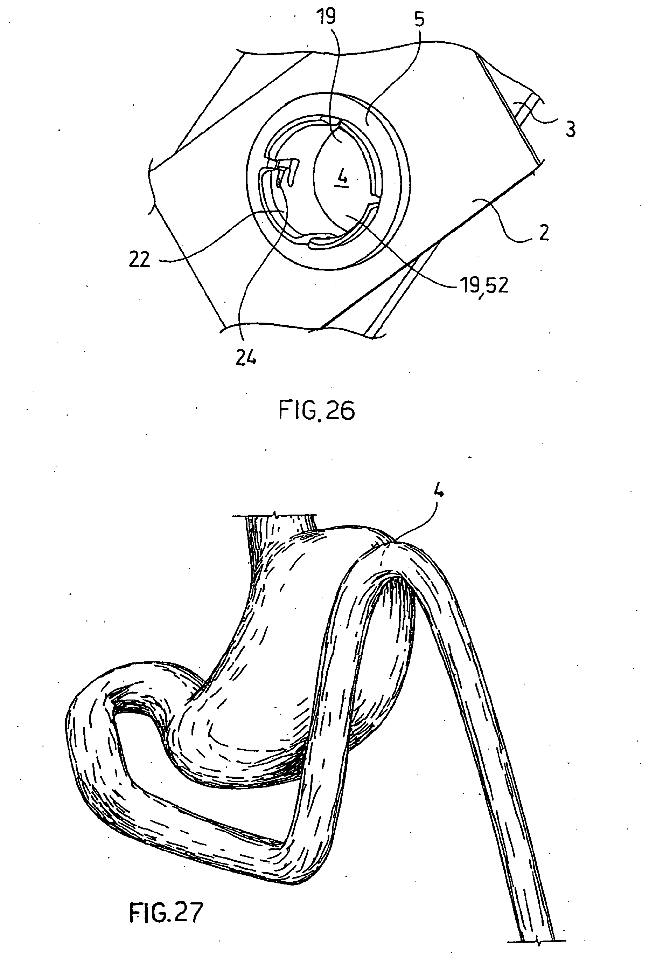

After the connection of the compression rings 5, 6 the balloon dilator 37 is deflated and both the balloon dilator and the anastomotic applier 28 are withdrawn from the body of the patient. Also after the definite connection of the compression rings 5, 6, the carrier member 7 is detached from the proximal compression ring 5 and removed, preferably together with the guide wire 10, by pulling the proximal guide wire end 10' in proximal direction. As the proximal ring 5 is definitely locked to the distal ring 6, during proximal traction of the guide wire 10 the carrier member 7 (which received the proximal ring by a weaker connection, e.g. by friction fit) detaches from the proximal compression ring 5 which rests connected to the distal compression ring 6 (Fig. 26, 27).

As has already been evidenced, it is preferred that the compression rings 5, 6, the carrier member 7, the locking device 20 as well as the anastomotic applier 28 and the balloon dilator 37 are guided from outside the body to the anastomotic site 4 by means of the same single guide wire 10.

Even though the exemplary method of performing an anastomosis has been described and illustrated with reference to a gastro-jejunostomy which is performed starting from the jejunum, such a gastro-jejunostomy might also be performed starting from the stomach. In this case, it might be advantageous to introduce a shielding device into the jejunum in order to prevent piercing of the intestine wall opposite the one intended to be anastomized.

Possibly, the tightness of the anastomosis can be tested using methylene blue.

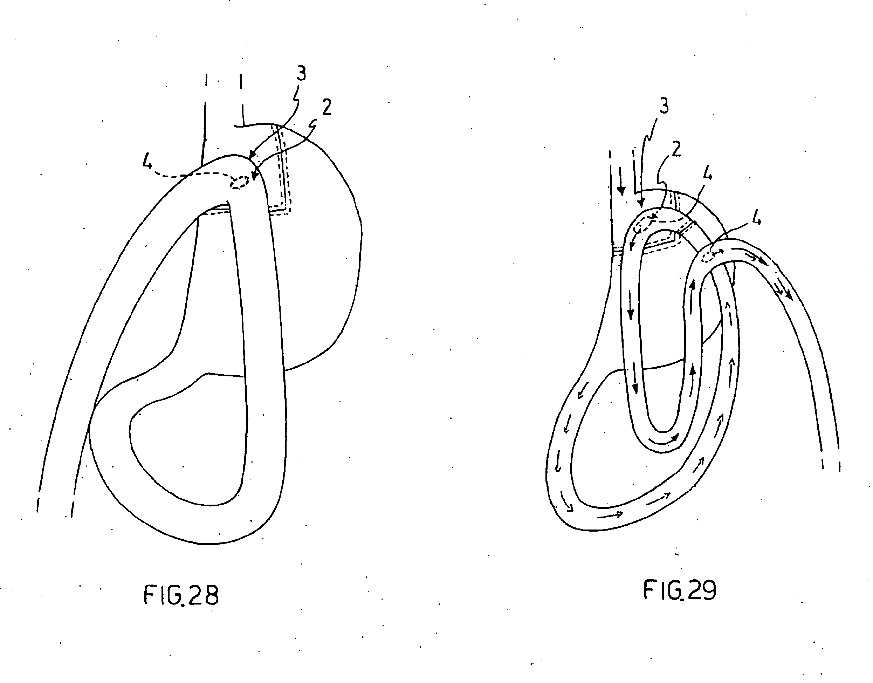

With reference to the annexed figures, the above described exemplary method can be advantageously performed endolumenally or partially endolumenally during a gastro-jejunostomy (G-J). In case the above described exemplary method forms part of a procedure for creating a gastrointestinal bypass, it is possible to proceed with a performance of a further anastomosis involving the small intestine (e.g. a jejuno-jejunostomy or an ileo-jejunostomy), possibly by applying the above described steps in analogous manner also to the further anastomosis (Fig. 28, 29).

From the foregoing description of the general steps of the exemplary method and of their specific application to a gastro-jejunostomy, those skilled in the art will appreciate that the exemplary method enables a continuous control of the positioning of the single parts of the anastomotic device 1 both during delivering to the anastomotic site and during deployment. The exemplary method further consents to perform the anstomosis endoluminally even though laparoscopic o partially laparoscopic steps in assisting and performing the anastomosis are not excluded.

The described exemplary method, particularly the use of a carrier member acting as adapter between the guide wire means and the anastomotic compression ring and the use of preferably only one single guide wire is not only advantageous for gastro-jejunostomy and jejuno-jejunostomy, but also for performing for example colo-proctostomy, jejuno-colostomy or anastomoses involving the Chole duct.

In the following, a set of surgical instruments will be described which has been especially developed and adapted for performing an anastomosis in accordance with the proposed exemplary method.

The instrumentation comprises advantageously one or more of the following components:
- the proximal and distal anastomotic rings 5, 6;
- the fixation needles 18;
- the carrier member 7;
- the locking device 20;
- the insertion device 39;
- the grasping device 45;
- the anastomotic applier 28;
- the balloon catheter 47;
- the balloon dilator 37;
- the guide wire 10;
- the snare 48,
which will be in the following described by means of illustrative embodiments.

### Detailed description of the anastomotic ring device

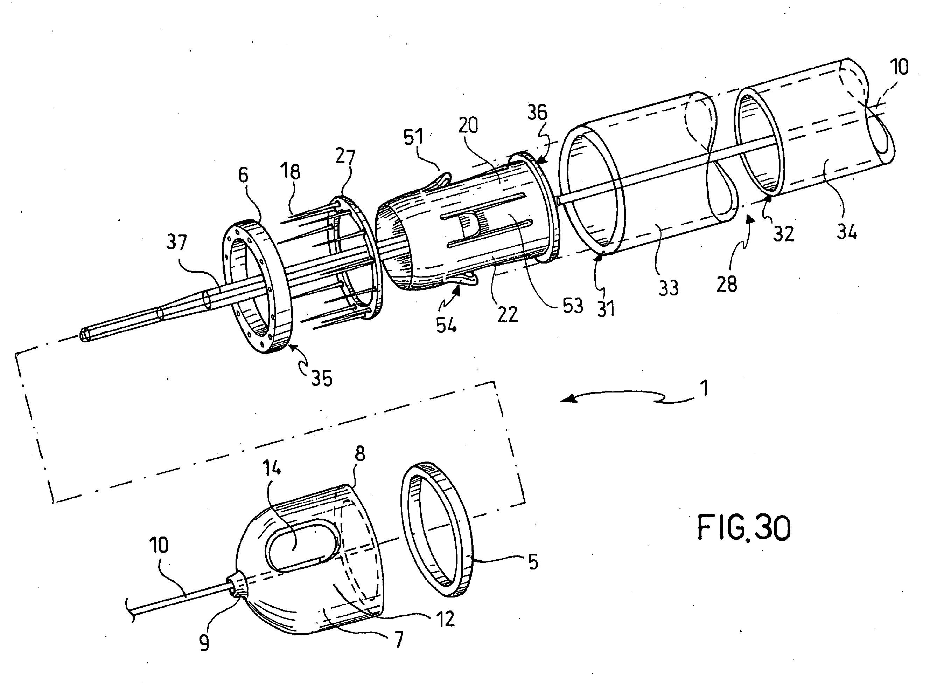

Figure 30 is an exploded view of an anastomotic device 1 with the proximal ring 5 and the distal ring 6 which is not only but particularly adapted to perform a gastro-jejunostomy according to the previously described exemplary method. The proximal compression ring 5 (or "bowel ring" or "intestinal ring" 5) is adapted to bear against the proximal tissue portion 2 (jejunal tissue), while the distal compression ring 6 (or "gastric ring" 6) is adapted to bear against the distal tissue portion 3 (gastric tissue) opposite the proximal tissue portion 2 and the proximal ring 5.

The proximal and distal rings 5, 6 are adapted to be latched together in at least one, preferably two or more locking positions corresponding to different compression rates acting on the tissue portions 2, 3 clamped between the rings.

Both the proximal 5 and distal compression ring 6 have a preferably closed circular annular shape (Fig. 30) in order to enable their mutual alignment and connection independently from their relative angular position.

The pressure surfaces 16, 17 of the proximal and distal rings 5, 6 destined to contact and clamp the proximal and distal tissue portions 2, 3 are substantially plane (fig. 30) or circumferentially wavy in order to increase the circumference of the anastomotic lumen with respect to the external ring circumference (not shown in the figures). Advantageously, the pressure surfaces 16, 17 are roughened or locally profiled in order to prevent the tissue 2, 3 to squeeze radially out of the ring device in response to the axial pressure.

In accordance with a preferred embodiment, the distal compression ring 6 (gastric ring) is adapted to be transported along the guide wire means 10 and comprises a shape or a specific interaction portion suitable to be engaged (e.g. by snap-fit, friction-fit, shape-fit or press-fit) by the anastomotic applier 28 and pushed by the latter along the guide wire 10.

Both compression rings 5, 6 are advantageously made of bio-absorbable or bio- fragmentable material.

### Detailed description of the fixation needles 18

In order to provide an additional fixation of the tissue portions 2, 3 to the compression rings 5, 6 to prevent the tissue from slipping out of engagement with the ring device, a group of needles 18 or staples is provided which is anchored in at least one of the compression rings and adapted to pierce through the tissue clamped therebetween.

The needles 18 can be directly fixed to a compression ring or to the locking device 20 or, as shown in figure 30, the group of needles 18 comprise a self supporting needle ring 27 which can be e.g. received by an apposite seat defined at the locking device 20 in order to anchor the needles 18 reliably to the ring device during and after its deployment.

Both the needles and the needle ring are advantageously but not necessarily made of bio-absorbable or bio-fragmentable material.

### Detailed description of the carrier member 7

The carrier member 7 is preferably distinct from the proximal and distal rings 5, 6 and comprises a first seat 8 adapted to receive the proximal compression ring 5 and a second seat 9 adapted to receive the guide wire 10, wherein means are provided for locking the guide wire 10 substantially non-relocatably to the second seat 9 so that the guide wire 10 can drag the carrier member 7 (which in turn carries the proximal compression ring 5) up to the proximal tissue portion 2. In this way the carrier member 7 acts as an adapter and connector between the proximal compression ring 5 and the guide wire 10, thereby allowing the proximal compression ring 5 to be designed and shaped predominantly for the specific requirements of the anastomosis, while the particular requirements and constraints of the ring delivery and positioning during the anastomosis are taken into account by the special design of the carrier member 7. As can be seen in the figures, thanks to the adapter feature of the carrier member 7, the proximal compression ring 5 can be embodied without any bulky seat for receiving the guide wire means.

Moreover, the provision of a distinct carrier member 7 makes it possible to use a centrally positioned single guide wire 10 whose central positioning with respect to the ring device doesn't cause problems in the ring design since there is no need to directly connect the proximal ring 5 to the guide wire 10.

According to a preferred embodiment, the first 8 and second seat 9 of the carrier member 7 are substantially coaxial to another so that the proximal compression ring 5 received by the first seat 8 is substantially coaxial with the guide wire 10 received by the second seat 9.

In the embodiments shown e.g. in figures 20, 30 and 31, the first seat 8 of the carrier member 7 has a substantially annular shape in order to directly engage the annular proximal compression ring 5, thereby obviating the need to adapt the shape of the compression ring to the guide wire connection requirements. Preferably, the first seat 8 is annular and generally plane (except of a local profiling provided for engaging the proximal compression ring 5). Alternatively, e.g. if the compression rings are wavy, the first seat 8 might also be wavy.

The second seat 9 of the carrier member 7 comprises a through hole or canal 11 substantially concentric to the first seat 8 and adapted to receive the guide wire 10.

In order to provide a free operational space on both sides of the anastomosis the second seat 9 is axially (proximally) spaced from the first seat 8 and, hence, from the proximal compression ring 5. In order to provide this axial distance between the two seats 8, 9 the carrier member 7 comprises a tubular or cup-shaped or, preferably, dome shaped side wall 12 having an annular distal base 13 forming the first seat 8 and an opposite proximal vertex 49 forming the second seat 9. Similarly, if the side wall 12 is cup shaped the first seat is formed at the distal open end and the second seat 9 is formed in a bottom portion of the cup shaped side wall 12.

Advantageously, the side wall 12 is not a continuous shell but it defines apertures 14 or it is embodied by a lattice or grid made of single rod portions forming a framework. This enables gastric and intestinal fluids and contents to pass through the side wall 12 without hindering the movement of the carrier member together with the proximal ring along the natural duct. Moreover, the window openings 14 contribute to a reduction of weight of the carrier member and to a visualization of its inside from outside the lateral wall 12, if necessary.

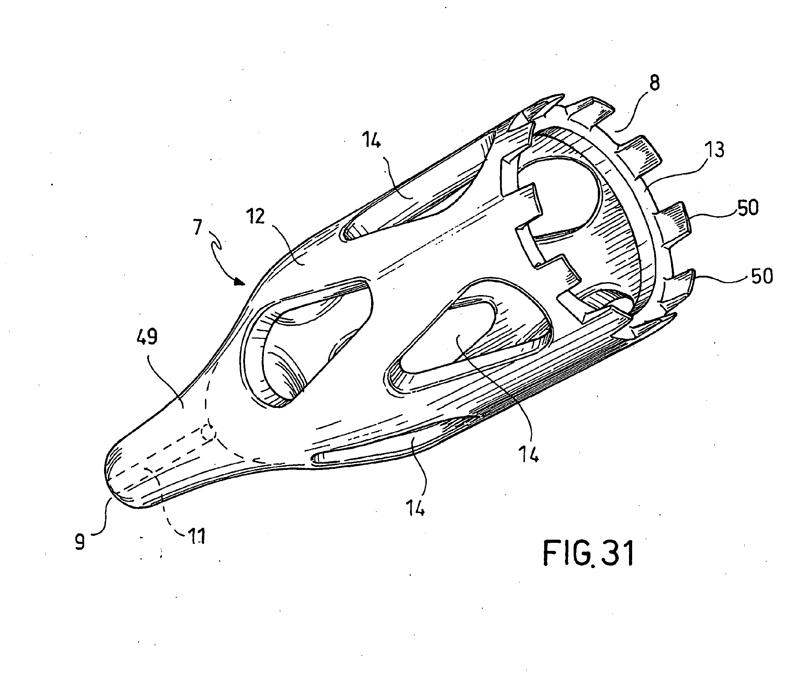

The first seat 8 of the carrier member 7 is adapted to detachably engage the proximal compression ring 5 in order to allow them to be separated after deployment of the compression ring. This detachable coupling can be obtained e.g. by complementary interacting snap - fit, friction - fit, shape - fit or press - fit features provided on both the first seat 8 and the proximal compression ring 5. In the embodiment shown in figure 31, the second seat 8 comprises a plurality of distally protruding snap or friction teeth 50 adapted to frictionally or snap engage the outer circumference of the proximal compression ring 5 in order to hold the latter during delivering and deployment.

The second seat 9 of the carrier member 7 can be plastically deformable such that it is tightened and pressed around the guide wire 10 in order to provide a press-fit connection between the carrier member 7 and the guide wire 10. Alternatively, the second seat 9 and a corresponding portion of the guide wire 10 have at least partially complementary shapes in order to provide a shape lock preventing the carrier member 7 from sliding along the guide wire 10.

In accordance with a preferred embodiment, distinct locking elements 15, e.g. clips or spacers, are provided which can be attached on the guide wire 10 on one or both sides of the second seat 9 in order to prevent the carrier member 7 from sliding along the guide wire 10.

Advantageously, the second seat 9 of the carrier member 7 is shaped to align the carrier member 7 with respect to the longitudinal guide wire direction. This alignment can be obtained by providing a sufficiently long (tubular) canal 11, so that the carrier member 7 is elastically axially oriented by the elastic bending resistance of the guide wire 10 passing through that canal 11.

### Detailed description of the locking device 20

Turning now to the locking device 20, in accordance with a first embodiment illustrated in figure 30, the locking device 20 comprises an annular proximal shoulder 25 with a distal engaging surface 21 suitable to engage the proximal end surface of the gastric compression ring 6 and a tubular longitudinal portion 22 which protrudes distally from the proximal shoulder 25. The longitudinal portion 22 has a shape adapted to be inserted through the central opening 52 of the gastric ring 6 and through the central opening 19 of the intestinal ring 5 and forms one or more, preferably four, elastically supported snapper teeth 51 adapted to snap engage the distal surface of the intestinal compression ring 5 (while the terms "proximal" and "distal" are generally referred to the direction along the guide wire loop, in the description of the snapper part 20 these terms refer to a surgeons point of view who applies the snapper part). According to the embodiment (Fig. 30) The longitudinal portion 22 comprises elastic beams 53 supporting the snapper teeth 51 and defining a sloped deviating surface 54 configured to slide, during insertion of the locking device 20, over an internal edge of the intestinal compression ring 5, thereby urging the elastic beams 53 elastically inward so that the snapper teeth 51 automatically snap engage the distal surface of the intestinal compression ring 5 by an elastically biased radially outward movement.

The provision of a distinct locking device allows the compression rings to be designed specifically to meet the requirements of the delivery and anastomosis, thereby avoiding compromises between these requirements and those of the connection of the compression rings. Advantageously, the outer diameter of the longitudinal portion 22 corresponds substantially (apart from the clearance necessary to allow its insertion) to the diameter of the central openings 19, 52 of the compression rings. The tubular shape of the locking device 20 stabilizes the shape of the anastomotic lumen and provides a protective lining which isolates the tissue portions 2, 3 clamped between the compression rings from fluids and contents passing through the anastomotic lumen at least during the period of wound healing.

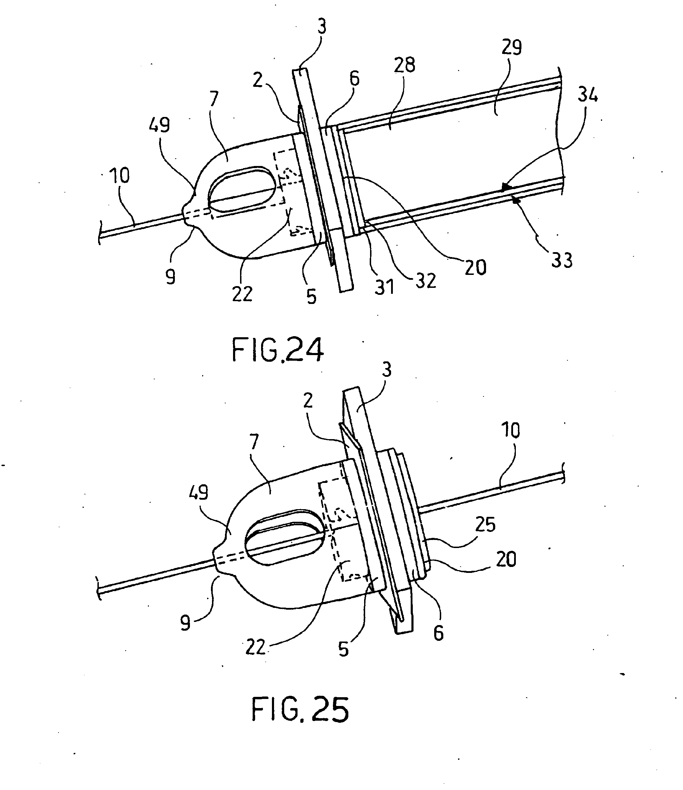

According to a further embodiment (Fig. 20 to 26) the longitudinal portion 22 defines protuberances or recesses 23 adapted to snap engage corresponding recesses or protuberances 24 provided at the intestinal compression ring 5. The recesses 23 of the locking device 20 comprise preferably one or more grooves defining a succession of one or more undercuts or hooks 26 in a manner to enable a progressive approximation of the locking device 20 together with the gastric ring 6 towards the intestinal ring 5 and to prevent them from moving apart. The above mentioned protuberances of the intestinal ring 5 comprise e.g. two or more teeth 24 protruding radially inside the central opening 19 of the intestinal compression ring 5 (Fig. 20).

The locking device 20 is advantageously made of bio-absorbable or bio- fragmentable material.

### Detailed description of the insertion device 39

The insertion device 39 (Fig. 2 to 4) comprises a flexible elongate insertion shaft preferably but not necessarily embodied by a visualization device 42 such as a gastroscope. The insertion device 39 defines internally at least one working or guide wire canal 40 adapted to slidably receive the guide wire 10. The guide wire canal 40 extends substantially along the entire length of the elongate insertion shaft and into an opening defined in a distal end 41 of the insertion device 39 in order to allow the guide wire to exit distally from the distal end 41.

### Detailed description of the grasping device 45

In accordance with an embodiment, the grasping device 45 comprises a visualization device such as a gastroscope provided with a working canal suitable to receive the balloon catheter 47 and the snare 48 in order to deliver them from outside the body to the operational site.

The insertion device 39 and the grasping device 45 are preferably but not necessarily the same instrument, e.g. a gastroscope.

### Detailed description of the anastomotic applier 28

Figures 20 to 24 and figure 30 show very simplified schematic illustrations of the anastomotic applier 28. The anastomotic applier 28 is preferably suitable to slide along the guide wire means 10 and comprises an elongate insertion shaft 29, e.g. a visualization device such as a gastroscope as well as a head portion adapted to be detachably connected with a distal end of the visualization device (the terms "distal" and "proximal" are here referred to the surgeons point of view). The head portion of the anastomotic applier 28 comprises a first pushing surface 31 adapted to perform a distal pressure on the gastric ring 6 and a second pushing surface 32 (preferably but not necessarily distinct from the first pushing surface 31) adapted to perform a distal pressure on the locking device 20 of the anastomotic device 1.

In acordance with an embodiment (Fig. 30), the first pushing surface 31 is formed by an annular distal end of a first tubular portion 33 and the second pushing surface 32 is formed by an annular distal end of a second tubular portion 34, wherein both tubular portions 33, 34 are coaxial and slidably arranged one inside the other. The first annular pushing surface 31 has a greater diameter than the second annular pushing surface 32 and is adapted to press against a proximal annular rim 35 of the gastric ring 6, while the second pushing surface 32 is adapted to press against a proximal annular rim 36 of the locking device 20, the rim 36 of the locking device being arranged radially inside the rim 35 of the gastric ring 6.

### Detailed description of the balloon dilator 37

The balloon dilator 37 is provided to widen the anastomotic passage openings 46, 44 in the tissue portions 2, 3 prior to completion of the anastomosis and to guide the gastric ring 6 and the locking device 20 towards the intestinal ring 5. The balloon dilator 37 has a central passage opening which makes it possible to insert the balloon dilator 37 over the guide wire 10 and to slide it along the latter to the operational site.

The balloon catheter 47 and the balloon dilator 37 are preferably but not necessarily the same device.

As can be readily appreciated from the foregoing description, the proposed exemplary method and, particularly, the proposed anastomotic device 1, the carrier member and the kit of surgical instruments allow to perform anastomoses of the digestive tract endoluminally even though their use in other techniques such as partial or pure laparoscopic approaches or traditional approaches involving one or more steps of open surgery, is possible and advantageous. Thanks to the carrier member it becomes possible to perform a guide wire loop with only one single guide wire and to simplify the shape and reduce the weight of the anastomotic compression rings, thereby reducing the invasiveness of the bypass-operation and improving the post-operative course of the patient.

Even though the function of the carrier member has been described in relation with an anastomotic ring device, which is its preferred use, the carrier can be also advantageously used (and the second seat thereof can be adapted to fit) for delivering surgical instruments and devices different from anastomotic rings to an operational site. In any case, the carrier member, acting as an adapter, obviates the need to adapt the shape of the instrument or device to the connection with the guide wire.

Although preferred embodiments of the invention have been described in detail, it is not the intention of the applicant to limit the scope of the claims to such particular embodiment, but to cover all modifications and alternative constructions falling within the scope of the invention.

## Claims

1. A carrier member (7) adapted to transport an anastomotic ring (5) endoluminally to an operational site (2) in the body of a patient, said carrier member (7) comprising:
- a first seat (8) configured to connect to said anastomotic ring (5);
- a second seat (9) for the connection of the carrier member (7) to a guide wire (10) to enable the carrier member (7) to be dragged by the guide wire 10 to said operational site (2),
**characterized in that:**
- said carrier member (7) comprises a side wall (12) defining a distal cavity adapted to allow an at least partial insertion of a surgical instrument, particularly a balloon dilator (37), during deployment of said anastomotic ring (5),
- said first seat (8) is formed along an annular base (13) at a distal open end of said side wall (12),
- said second seat (9) is formed at a vertex (49) of the side wall (12) opposite its base (13), so that said first seat (8) and said second seat (9) are axially spaced apart.

2. A carrier member (7) according to claim 1, wherein said first seat (8) and said second seat (9) are substantially coaxial.

3. A carrier member (7) according to any one of the preceding claims, comprising a substantially cup-shaped or dome-shaped side wall (12), said first seat (8) being formed along a larger base (13) of said side wall (12) and said second seat (9) being formed at a vertex (49) or bottom of the side wall (12) opposite its base (13),

4. A carrier member (7) according to the preceding claim, wherein said side wall (12) defines one or more window openings (14) adapted to allow fluids and contents to pass through the side wall (12) during dragging.

5. A carrier member (7) according to any one of the preceding claims, wherein said first seat (8) of the carrier member (7) is configured to detachably connect said surgical device, particularly said anastomotic ring (5) to allow their separation after completion of the deployment.

6. A carrier member (7) according to any one of the preceding claims, wherein said first seat (8) comprises snap-fit or friction-fit or press-fit features to connect the surgical device, particularly the anastomotic ring (5).

7. A carrier member (7) according to the preceding claim, wherein the first seat (8) comprises a plurality of distally protruding snap or friction teeth (50) adapted to frictionally or snap engage the outer circumference of the surgical device, particularly the anastomotic ring (5).

8. A carrier member (7) according to any one of the preceding claims, wherein said second seat (9) comprises a hole or canal (11) substantially coaxial to said first seat (8) and suitable to receive said guide wire (10), in which said hole or canal (11) is sufficiently long to realize an elastic axial alignment of the carrier member (7) with respect to the guide wire (10).

9. A carrier member (7) according to any one of the preceding claims, wherein the second seat (9) is lockable to the guide wire (10) by means of:
- a portion of the seat (9) which is deformable or displaceable in a manner to tighten the second seat around the guide wire (10) and/or
- one or more locking elements (15) which can be attached to the guide wire (10) and which are configured to interfere with the second seat (9) in a manner to prevent the carrier member (7) from sliding along the guide wire (10).

10. An anastomotic device (1) for performing an endoluminal or transluminal anastomosis, such as in a gastro-jejunostomy, in a jejuno-jejunostomy, in a coloproctostomy, in a jejuno-colostomy or anastomoses of the chole duct, comprising;
- a proximal ring (5) and a distal ring (6) intended to be positioned on both sides of a proximal and distal tissue portion (2, 3) to approximate and clamp the tissue portions (2, 3) between them;
- a carrier member (7) according to any one of the preceding claims, distinct from the proximal and distal rings (5, 6), wherein said first seat (8) is configured to connect to said proximal ring (5).

11. An anastomotic device (1) according to the preceding claim, further comprising a locking device (20) having an engaging surface (21) adapted to engage the distal ring (6) and a longitudinal portion (22) adapted to be inserted in the central openings of the distal and proximal rings (5, 6) and to snap engage the proximal ring (5) to latch the rings (5, 6) together, wherein the 15 carrier member (7) and the longitudinal portion (22) of the locking device (20) are configured such that they do not interfere during the insertion and snap engagement of the locking device (20) in the rings (5, 6).

## Patentansprüche

1. Ein Trägerglied (7), das für den endoluminalen Transport eines anastomotischen Rings (5) bis zu einer Operationsstelle (2) im Körper eines Patienten eingerichted ist, wobei das Trägerglied (7) Folgendes umfasst:
- einen ersten Sitz (8), der für die Verbindung mit dem anastomotischen Ring (5) gebaut worden ist;
- einen zweiten Sitz (9) für die Verbindung des Trägerglieds (7) mit einem Führungsdraht (10), damit das Trägerglied (7) durch den Führungsdraht (10) zur Operationsstelle (2) gezogen werden kann,
**dadurch gekennzeichnet dass**:
- das Trägerglied (7) eine Seitenwand (12) umfasst, die einen distalen Hohlraum aufweist, der dazu geeignet ist, eine zumindest partielle Einführung eines chirurgischen Geräts, im Besonderen eines Ballondilatators (37), während der Implantation des anastomotischen Rings (5) zu ermöglichen,
- der erste Sitz (8) lang einer annularen Basis (13) bei einem distalen offenen Ende der Seitenwand (12) gebildet ist,
- der zweite Sitz (9) an einer Spitze (49) der Seitenwand (12) seiner Basis (13) gegenüberliegend gebildet ist, so dass der erste Sitz (8) und der zweite Sitz (9) in axialer Richtung auseinander liegen.

2. Ein Trägerglied (7) nach Anspruch 1, wobei der erste Sitz (8) und der zweite Sitz (9) im Wesentlichen koaxial sind.

3. Ein Trägerglied (7) nach einem der vorhergehenden Ansprüche, das eine im Wesentlichen Tassenförmige oder Kuppelförmige Seitenwand (12) umfasst, wobei der erste Sitz (8) lang einer breiteren Basis (13) der Seitenwand (12) gebildet ist und der zweite Sitz (9) an einer Spitze (49) oder Boden der Seitenwand (12) seiner Basis (13) gegenüberliegend gebildet ist.

4. Ein Trägerglied (7) nach dem vorhergehenden Anspruch, wobei die Seitenwand (12) eine oder mehrere Fensteröffnungen (14) aufweist, die dazu geeignet sind, Flüssigkeiten oder Inhaltstoffe während des Ziehens durch die Seitenwand (12) gleiten zu lassen.

5. Ein Trägerglied (7) nach einem der vorhergehenden Ansprüche, wobei der erste Sitz (8) des Trägerglieds (7) so ausgebildet ist, dass er mit dem chirurgischen Gerät lösbar verbunden ist, im Besonderen mit dem anastomotischen Ring (5), um deren Trennung nach der Beendigung der Aufdehnung zu ermöglichen.

6. Ein Trägerglied (7) nach einem der vorhergehenden Ansprüche, wobei der erste Sitz (8) Schnappeinbau-, Reibungspassung- oder Presssitzmerkmale aufweist, um eine Verbindung mit dem chirurgischen Gerät zu haben, im Besonderen mit dem anastomotischen Ring (5).

7. Ein Trägerglied (7) nach dem vorhergehenden Anspruch, wobei der erste Sitz (8) eine Vielzahl an distal herausragenden Schnapp- oder Reibungszähnen (50) aufweist, die dazu geeignet sind, den äußeren Umfang des chirurgischen Geräts durch Reibung oder Schnappen zu greifen, im Besonderen des anastomotischen Rings (5).

8. Ein Trägerglied (7) nach einem der vorhergehenden Ansprüche, wobei der zweite Sitz (9) einen Hohlraum oder Kanal (11) umfasst, der im Wesentlichen koaxial zum ersten Sitz (8) ist und dazu geeignet ist, den Führungsdraht (10) aufzunehmen, wobei der Hohlraum oder Kanal (11) lang genug ist, um eine elastische axiale Ausrichtung des Trägerglieds (7) in Bezug auf den Führungsdraht (10) herzustellen.

9. Ein Trägerglied (7) nach irgendeinem der vorhergehenden Ansprüche, wobei der zweite Sitz (9) am Führungsdraht (10) verriegelbar ist, mit Hilfe von Folgendem:
- einem Teil des Sitzes (9), der auf solch eine Weise verformbar oder verschiebbar ist, dass der zweite Sitz um den Führungsdraht (10) festgezogen wird, und/oder
- einem oder mehreren Verschlussbestandteilen (15), die an den Führungsdraht (10) angebracht werden können und die so gestaltet sind, dass sie mit dem zweiten Sitz (9) so interferieren, dass das Trägerglied (7) am Gleiten entlang des Führungsdrahts (10) verhindert wird.

10. Ein anastomotisches Gerät (1) zur Durchführung einer endoluminalen oder transluminalen Anastomose, wie bei einer Gastrojejunostomie, Jejunojejunostomie, Koloproktostomie, Jejunokolostomie, oder bei einer Anastomose des Gallengangs, wobei das Gerät Folgendes umfasst:
- einen proximalen Ring (5) und einen distalen Ring (6), die an beiden Seiten eines proximalen und distalen Gewebeteils (2, 3) anzubringen sind, um die Gewebeteile (2, 3) untereinander anzunähern und zu verklemmen;
- ein Trägerglied (7) nach einem der vorhergehenden Ansprüche, das von dem proximalen und distalen Ring (5, 6) getrennt ist, wobei der erste Sitz (8) so gestaltet ist, dass er mit dem proximalen Ring (5) in Verbindung kommt.

11. Ein anastomotisches Gerät (1) nach dem vorhergehenden Anspruch, das weiters eine Sperre (20) mit einer Eingriffoberfläche (21) umfasst, die dazu geeignet ist, den distalen Ring (6) und einen längsgerichteten Teil (22) ineinander greifen zu lassen, der dazu geeignet ist, in die zentralen Öffnungen des distalen und proximalen Rings (5, 6) eingefügt zu werden und den proximalen Ring (5) durch Schnappen zu greifen, um die Ringe (5, 6) miteinander einzuklinken, wobei das Trägerglied (7) und der längsgerichtete Teil (22) der Sperre (20) so gestaltet sind, dass sie während der Einführung und dem Scnappeingreifen der Sperre (20) in die Ringe (5, 6) miteinander nicht interferieren.

## Revendications

1. Elément de support (7) adapté pour transporter un anneau anastomotique (5) de manière endoluminale à un site opératoire (2) dans le corps d'un patient, ledit élément de support (7) comprenant :
- un premier siège (8) configuré pour se connecter audit anneau anastomotique (5) ;
- un deuxième siège (9) pour la connexion de l'élément de support (7) à un fil de guidage (10) pour permettre au fil de guidage (10) d'entraîner l'élément de support (7) vers le site opératoire (2),
**caractérisé en ce que** :
- ledit élément de support (7) comprend une paroi latérale (12) définissant une cavité distale adaptée pour permettre une insertion au moins partielle d'un instrument chirurgical, en particulier un ballon dilatateur (37), durant le déploiement dudit anneau anastomotique (5),
- ledit premier siège (8) est formé suivant une base annulaire (13) à une extrémité distale ouverte de ladite paroi latérale (12),
- ledit deuxième siège (9) est formé à un sommet (49) de la paroi latérale (12) opposé à sa base (13), de manière que ledit premier siège (8) et ledit deuxième siège (9) soient écartés axialement.

2. Elément de support (7) selon la revendication 1, dans lequel ledit premier siège (8) et ledit deuxième siège (9) sont sensiblement coaxiaux.

3. Elément de support (7) selon l'une quelconque des revendications précédentes, comprenant une paroi latérale en forme de coupelle ou en forme de coupole (12), ledit premier siège (8) étant formé le long d'une base plus grande (13) de ladite paroi latérale (12) et ledit deuxième siège (9) étant formé à un sommet (49) ou un fond de la paroi latérale (12) opposé à sa base (13).

4. Elément de support (7) selon la revendication précédente, dans lequel la paroi latérale (12) définit une ou plusieurs fenêtres d'ouverture (14) adaptées pour permettre le passage de fluides et contenus à travers la paroi latérale (12) durant l'entraînement.

5. Elément de support (7) selon l'une quelconque des revendications précédentes, dans lequel ledit premier siège (8) de l'élément de support (7) est configuré pour connecter de manière amovible ledit dispositif chirurgical, en particulier ledit anneau anastomotique (5), pour permettre leur séparation après achèvement du déploiement.

6. Elément de support (7) selon l'une quelconque des revendications précédentes, dans lequel ledit premier siège (8) comprend des moyens d'insertion à déclic, friction ou à force pour connecter ledit dispositif chirurgical, en particulier l'anneau anastomotique (5).

7. Elément de support (7) selon l'une quelconque des revendications précédentes, dans lequel le premier siège (8) comprend une pluralité de dents de friction ou pression faisant saillie de manière distale (50) adaptées pour engager à déclic ou par friction la circonférence extérieure du dispositif chirurgical, en particulier l'anneau anastomotique (5).

8. Elément de support (7) selon l'une quelconque des revendications précédentes, dans lequel ledit deuxième siège (9) comprend un trou ou canal (11) sensiblement coaxial avec ledit premier siège (8) et adapté pour recevoir ledit fil de guidage (10), dans lequel ledit trou ou canal (11) est suffisamment long pour réaliser un alignement axial élastique de l'élément de support (7) par rapport au fil de guidage (10).

9. Elément de support (7) selon l'une quelconque des revendications précédentes, dans lequel le deuxième siège (9) est blocable avec le fil de guidage (10) au moyen de :
- une partie du siège (9) qui est déformable ou déplaçable de manière à serrer le deuxième siège autour du fil de guidage (10) et/ou
- un ou plusieurs éléments de blocage (15) qui peuvent être fixés au fil de guidage (10) et qui sont configurés pour interférer avec le deuxième siège (9) de manière à empêcher l'élément de support (7) de coulisser le long du fil de guidage (10).

10. Dispositif anastomotique (1) pour effectuer une anastomose endoluminale ou transluminale, telle que dans une gastro-jéjunostomie, dans une jéjuno-jéjunostomie, dans une colo-proctostomie, dans une jéjuno-colostomie ou des anastomoses du conduit chole, comprenant :
- un anneau proximal (5) et un anneau distal (6) destinés à être positionnés des deux côtés d'une portion de tissu proximale et distale (2, 3) pour approcher et serrer les portions de tissu (2, 3) entre elles ;
- un élément de support (7) selon l'une quelconque des revendications précédentes, distinct des anneaux proximal et distal (5, 6), dans lequel ledit premier siège (8) est configuré pour se connecter audit anneau proximal (5).

11. Dispositif anastomotique (1) selon la revendication précédente, comprenant en outre un dispositif de blocage (20) comportant une surface d'engagement (21) adaptée pour engager l'anneau distal (6) et une portion longitudinale (22) adaptée pour être insérée dans les ouvertures centrales des anneaux distal et proximal (5, 6) et pour engager à déclic l'anneau proximal (5) pour verrouiller les anneaux (5, 6) ensemble, dans lequel l'élément de support (7) et la portion longitudinale (22) du dispositif de blocage (20) sont configurés de manière qu'ils n'interfèrent pas durant l'insertion et l'engagement à déclic du dispositif de blocage (20) dans les anneaux (5, 6).
